# FASCICULE DE BREVET EUROPEEN

(11) **EP 1 605 839 B1**
(45) Date de publication et mention de la délivrance du brevet: **17.12.2008**
(21) Numéro de dépôt: 04706707.9
(22) Date de dépôt: 30.01.2004
(51) Int. Cl.: A61B 17/22

(54) **DISPOSITIF GENERATEUR D UNE ONDE DE CHOC A SIMPLE COUP**
VORRICHTUNG ZUR ERZEUGUNG EINER STOSSWELLE
SINGLE-BLOW SHOCKWAVE GENERATION DEVICE

(30) Priorité: 14.02.2003 FR 0301793
(43) Date de publication de la demande: 21.12.2005
(73) Titulaire: Duliver International Ltd., Road Town, Tortola (VG)
(72) Inventeur: Lebet, Alain, 1006 Lausanne (CH)
(74) Mandataire: Jacob, Reuben Ellis
(86) Numéro de dépôt international: PCT/FR2004/000208
(87) Numéro de publication internationale: WO 2004/078048

(56) Documents cités:
- EP-A- 1 163 882
- WO-A-95/22934
- WO-A-98/26705
- US-A- 4 016 873
- US-A- 4 716 890
- US-A- 5 160 336
- US-A- 5 906 623

## Description

L'invention concerne un dispositif générateur d'ondes de choc à simple coup et son procédé de mise en oeuvre.

Les dispositifs générateurs d'ondes de choc sont notamment utilisés en chirurgie urologique pour la désintégration de calculs des voies urinaires. Il existe deux principaux types de dispositifs générateurs d'ondes de choc: un premier type concernant des dispositifs générateurs d'ondes de choc extra corporels et un deuxième type concernant des dispositifs générateurs d'ondes de choc percutanés.

Les dispositifs de désintégration du premier type utilisent un générateur d'ondes de choc électrique ou piézo-électrique où le guide d'ondes de choc est constitué d'une poche contenant un liquide, en contact avec le corps du patient, coopérant avec un réflecteur ellipsoïdal qui, dans un premier temps, reçoit une onde de choc diffusée dans un large espace pour la focaliser suivant un cône très ouvert dont le sommet se trouve à l'intérieur du calcul urinaire à désintégrer de manière que l'onde de choc soit de faible amplitude lorsqu'elle traverse les tissus vivants afin de les léser le moins possible et qu'elle est une amplitude maximum lorsqu'elle est concentrée au sommet du cône.

Les dispositifs de désintégration du deuxième type utilisent, par exemple, des endoscopes spécialisés par rapport à la nature de l'intervention à effectuer en fonction de l'importance et de la position du calcul urinaire à évacuer. Lorsque le calcul est à l'intérieur du rein, l'endoscope est introduit par voie percutanée directement dans le rein et lorsque le calcul est dans l'uretère, il est préférable d'introduire l'endoscope par les voies naturelles via la vessie et de remonter dans l'uretère. L'onde de choc est transmise par un guide d'onde qui est une tige métallique de section circulaire d'un diamètre de l'ordre de dix à vingt dixièmes de millimètre acceptant une déformation élastique. Le guide d'onde de choc comporte une première extrémité où est générée l'onde de choc et une deuxième extrémité qui est appliquée contre le calcul.

Les dispositifs de désintégration du premier type ne permettent pas la production d'ondes de choc de forte amplitude à cause des risques de lésions des tissus vivants traversés. En conséquence, la désintégration d'un calcul nécessite une multitude de chocs qui réduisent le calcul en petits fragments évacuables par les voies urinaires.

Les dispositifs de désintégration du deuxième type existants, notamment celui décrit dans le brevet EP0317507, utilisent des trains d'ondes de choc de faible amplitude qui ont aussi pour caractéristique de briser le calcul à désintégrer en une multitude de fragments qui peuvent être éliminés par aspiration, lavage ou par évacuation par les voies naturelles.

L'élimination par les voies naturelles des fragments de calculs désintégrés est très douloureuse, ce qui incite à préférer l'élimination par lavage lorsqu'elle est possible suite à une intervention par endoscopie. Un inconvénient de ces modes d'élimination des calculs est qu'il reste toujours des débris de calculs qui ne sont pas éliminés et qui peuvent servir de base pour la formation de nouveaux calculs.

Des moyens d'étanchéification de pièces mobiles entre elles, par rapport aux gaz, utilisent généralement des joints toriques pour lesquels l'étanchéité est obtenue grâce à la coopération soit des cercles d'étanchéité supérieurs et inférieurs des joints toriques pincés entre deux surfaces planes, soit à la coopération entre un cercle d'étanchéité latéral interne et un cercle d'étanchéité latéral externe du joint torique pincé entre un alésage de révolution et un cylindre de révolution. Ces moyens d'étanchéification peuvent être classés, par exemple, en trois types de dispositifs : un dispositif d'étanchéité de premier type est constitué d'un joint torique positionné dans une gorge pratiquée dans un alésage et dont l'étanchéité est réalisée par la coopération entre le cercle d'étanchéité latéral interne et le cercle d'étanchéité latéral externe. Un dispositif d'étanchéité de deuxième type est constitué par un joint torique placé dans une gorge pratiquée dans un cylindre de révolution. Un dispositif d'étanchéité de troisième type est constitué d'un joint torique placé dans une gorge circulaire creusée dans une surface plane.

L'invention a pour objet un dispositif générateur d'une onde de forte amplitude transmise par voie percutanée ou naturelle comme défini dans la revendication 1, à travers un endoscope, permettant de réaliser une fragmentation de calcul urinaire controlée afin de le casser en un petit nombre de morceaux d'une dimension nécessaire et suffisante pour en permettre l'extraction manuelle à l'aide d'une pince à travers l'endoscope, mis en place pour la fragmentation, pour les visualiser, les saisir et les extraire.

L'invention sera mieux comprise par la description qui va suivre se rapportant aux dessins annexés sur lesquels :
La figure 1A représente en élévation un dispositif selon la version préférée de l'invention.
La figure 1B représente une coupe schématique du dispositif selon la version préférée de l'invention en ordre de marche.
La figure 1C représente la coupe schématique de la figure 1A après déclenchement d'un cycle de fonctionnement.
La figure 1D représente un détail de la figure 1C au moment de la génération d'une onde de choc.
Les figures 2A à 2D représentent chacune un détail de la figure 1B, l'ensemble de ces figures représentant la totalité de la figure 1B.

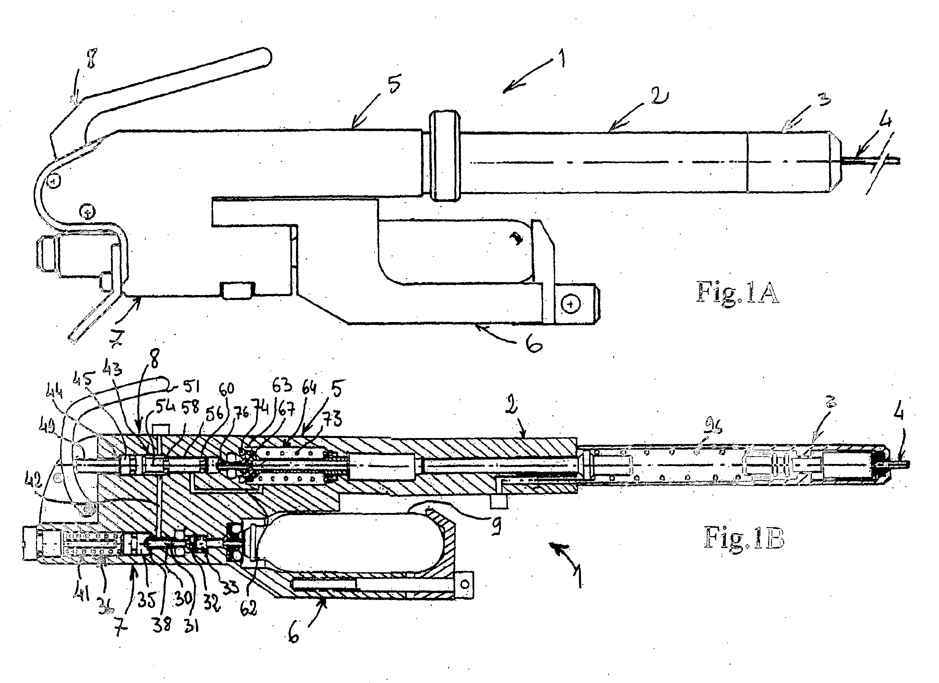

L'invention consiste en un dispositif générateur d'ondes de chocs mécaniques à simple coup 1 (fig.1A et fig.1B) comprenant des moyens de frappe 2 venant frapper à grande vitesse des moyens générateurs d'une onde de choc 3 qui est transmise par des moyens de transfert d'onde de choc 4 à un objet à désintégrer avec lequel lesdits moyens sont en contact direct ou indirect, les moyens de frappe 2 étant mis en mouvement grâce à la détente d'un gaz sous haute pression introduit, préalablement à chaque production d'une onde de choc, dans des moyens d'accumulation 5 alimentés en gaz à haute pression, à partir de moyens de stockage autonomes 6 de gaz sous très haute pression, grâce à des moyens de détente de gaz 7 et à des moyens d'alimentation et d'étanchéification; le gaz stocké dans les moyens d'accumulation 5 étant libéré par la manoeuvre manuelle de moyens de commande 8 qui, dans un premier temps, rend étanche aux gaz, par un moyen d'étanchéification, la communication des moyens de stockage autonomes 6 et de détente 7 d'une part, avec les moyens d'accumulation 5 d'autre part, puis, dans un deuxième temps, met en communication les moyens d'accumulation 5 et les moyens de frappe 2 ; le retour à la situation initiale des moyens de frappe 2 étant assuré par la libération d'énergie accumulée, par des moyens mécaniques, au cours du cycle de production de l'onde de choc, le retour à la situation initiale des moyens de commande 8 étant assuré par l'action de la haute pression des gaz restés au niveau des moyens de détente 7 et les moyens d'alimentation correspondants.

Le gaz utilisé est assimilable à un gaz parfait à la température d'utilisation qui est de l'ordre de vingt degrés centigrades dans une chambre d'accumulation, constituant un moyen d'accumulation, et à la pression d'utilisation qui est une haute pression de l'ordre de quinze à trente bars, et chimiquement compatible avec son lieu d'utilisation ; ce peut être, par exemple, de l'air ou de l'azote provenant d'une bouteille de gaz sous pression gonflée sous très haute pression à deux cents bars environ, constituant un moyen de stockage autonome d'une contenance allant d'un demi-litre à quelques litres ; elle est reliée au dispositif générateur d'onde de choc mécanique à simple coup 1 par une tuyauterie flexible et par l'intermédiaire d'un détendeur, constituant un moyen de détente, par exemple solidaire de la bouteille de gaz qui ramène la très haute pression de deux cents bars à une haute pression allant de quinze à trente bars.

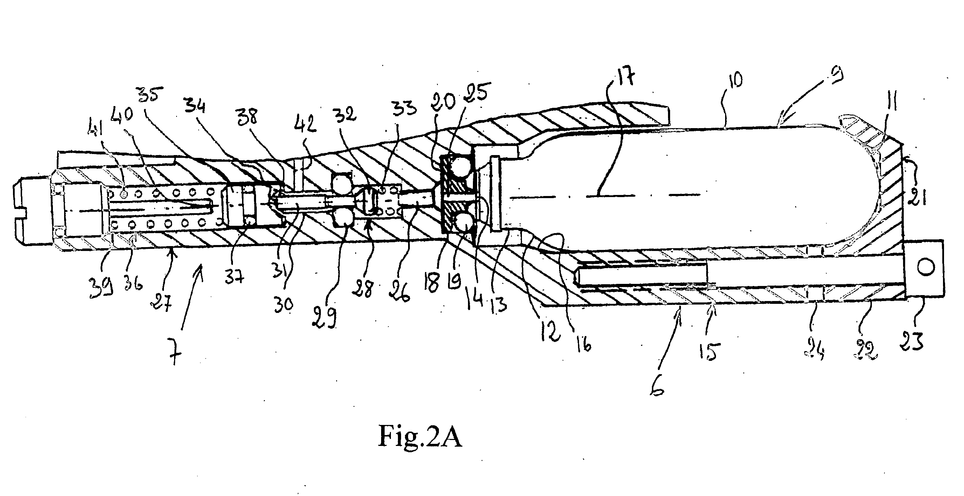

Dans une version préférée de l'invention, le gaz utilisé est, par exemple, du gaz carbonique qui est commercialisé dans un micro-conteneur de gaz 9 (fig.2A) à usage unique, constituant un moyen de stockage de gaz d'une contenance de l'ordre de deux centilitres à une pression de l'ordre de soixante dix bars à laquelle le gaz carbonique est liquide ce qui permet de stocker un volume de gaz carbonique important dans un petit volume de stockage ; la description qui suit peut s'appliquer à d'autres gaz tout en restant dans le cadre de l'invention.

Le micro-conteneur de gaz 9 est constitué d'un corps cylindrique 10 d'un diamètre extérieur de l'ordre de dix-huit millimètres dont une extrémité arrière 11 est fermée par une paroi hémisphérique et l'extrémité avant 12 est prolongée par un épaulement, puis par un goulot 13 comportant une partie latérale sensiblement cylindrique fermé par une capsule de fermeture 14, l'ensemble de l'épaulement et du goulot 13 ayant une longueur de l'ordre de treize millimètres et la longueur totale de l'ensemble étant de l'ordre de quatre-vingt millimètres ; le micro-conteneur de gaz 9 est directement intégré dans le dispositif générateur d'onde de choc mécanique à simple coup 1 : 1 il est placé dans un berceau 15 composé de deux demi-berceaux ; un demi-berceau avant 16 est constitué d'un alésage cylindrique de révolution, suivant un premier axe de symétrie 17, de diamètre légèrement supérieur à celui du corps du micro-conteneur de gaz 9 et comporte un fond muni d'un réceptacle calibré 18 pour recevoir le goulot 13 du micro-conteneur de gaz 9, la partie latérale étant équipée d'un premier dispositif d'étanchéité de premier type 19 par rapport à la partie latérale du goulot 13, tandis que la partie centrale du réceptacle calibré 18 comporte un dispositif de perforation 20 de la capsule de fermeture 14 ; le demi-berceau arrière 21 est composé d'un dispositif de maintien 22 du fond hémisphérique du micro-conteneur de gaz 9 centré sur le premier axe de symétrie 17 susceptible de coulisser, parallèlement à ce dernier, grâce à un étrier de guidage 24 et un dispositif de serrage 23 qui prend appui sur le demi-berceau avant 16 ; l'étrier de guidage comporte de larges ouvertures latérales permettant de glisser l'extrémité avant 12 du micro-conteneur de gaz 9 dans le réceptacle calibré 18 du demi-berceau avant 16, lorsque le demi-berceau arrière 21 est escamoté ; il suffit de faire coulisser le dispositif de maintien 22 qui vient en appui sur le fond hémisphérique 11 et d'effectuer le serrage en poussant le micro-conteneur de gaz 9 contre le dispositif de perforation 20 jusqu'à ce que la capsule de fermeture 14 soit perforée ; le dispositif de perforation 20 comporte un moyen de transfert du gaz ; le dispositif de perforation 20 est, par exemple, du type de ceux utilisés sur les bonbonnes de gaz butane à usage unique et un moyen de transfert de gaz est un alésage cylindrique central 25 permettant le passage du gaz extrait du micro-conteneur 9. Le dispositif de perforation 20 communique, par l'intermédiaire d'un premier conduit 26, avec un dispositif de détente 27 intégré au dispositif générateur d'ondes de choc mécanique à simple coup 1 constituant un moyen de détente de gaz 7 (fig.1A et fig. 1B) ; il est constitué, par exemple, d'une première chambre 28 (fig.2A) cylindrique de révolution dans la partie arrière de laquelle débouche le premier conduit 26 et dont la partie avant comporte un premier orifice circulaire bordé par un deuxième dispositif d'étanchéité de premier type 29 ; le premier orifice circulaire est prolongé par un deuxième conduit 30 dans lequel coulisse librement, pour permettre le passage du gaz, la queue de soupape 31 d'une soupape d'alimentation comportant une tête de soupape 32, située dans la première chambre 28, de diamètre sensiblement inférieur à celui de la première chambre 28, dont la partie inférieure comporte une surface annulaire d'étanchéité entourant la queue de soupape 31 ; la partie supérieure de la tête de soupape 32 est repoussée par un premier ressort taré 33 afin d'appuyer la surface annulaire d'étanchéité de la tête de soupape 32 sur le deuxième dispositif d'étanchéité de premier type 29 ; l'extrémité libre de la queue de soupape 31 coulisse dans un premier alésage 34, de profondeur déterminée, lui servant de guide, pratiqué dans la tête d'un premier piston cylindrique 35, dont le fond, du premier alésage 34, sert de repoussoir de la queue de soupape 31 ; le premier piston 35 coulisse dans une deuxième chambre 36 cylindrique de révolution qu'il sépare en un premier et un deuxième espace, de volume variable, étanches l'un par rapport à l'autre grâce à un premier dispositif d'étanchéité de deuxième type solidaire du premier piston 35 ; le deuxième conduit 30 débouche dans la partie arrière 38 de la deuxième chambre 36, délimitant partiellement le premier espace de la deuxième chambre 36 par un deuxième orifice circulaire permettant le passage libre de la queue de soupape 31 et mettant la deuxième chambre 36 en communication avec le circuit de gaz ; la partie arrière 38 de la deuxième chambre 36 sert de butée à la tête du premier piston 35 ; le deuxième espace, délimité partiellement par la partie avant 39 de la deuxième chambre 36, comporte une butée de piston 40, coaxiale avec celui de la deuxième chambre 36, limitant la course du premier piston 35 et servant de guide à un deuxième ressort taré 41.

L'ensemble composé par la première et la deuxième chambres 28 et 36, la soupape d'alimentation, le premier piston 35, les premier et deuxième ressorts tarés 33,41 constituent un moyen de détente de gaz 7 (fig.1A et fig.1B) permettant d'obtenir, dans le deuxième conduit 30 (fig.2A) de gaz entourant la queue de soupape 31, une pression nominale définie avec une bonne précision comprise entre quinze et trente bars.

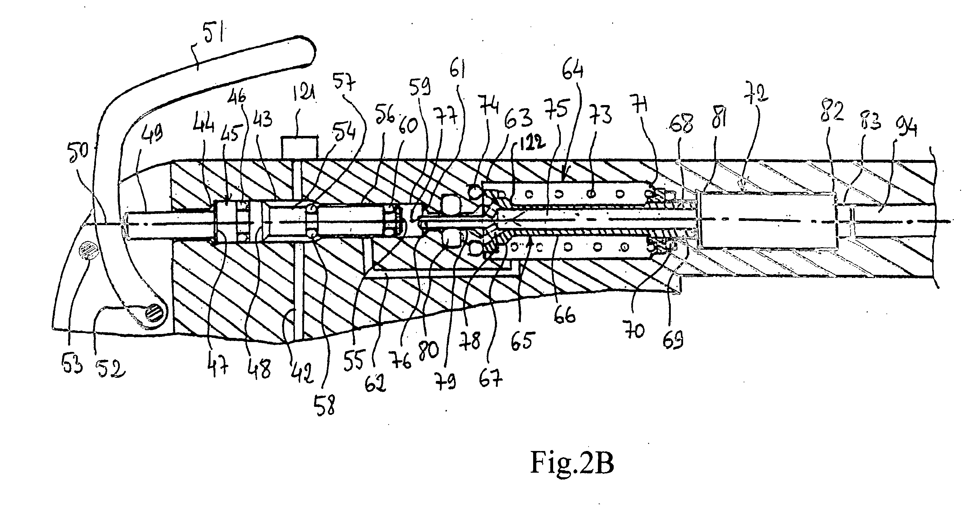

Un troisième conduit 42 (fig.2B) partant du deuxième conduit 30 débouche dans la paroi latérale d'une troisième chambre 43 cylindrique de révolution ; cette troisième chambre comporte une partie arrière 44 dans laquelle coulisse un deuxième piston 45 comportant un deuxième dispositif d'étanchéité de deuxième type 46 au gaz, un côté arrière 47 et un côté avant 48 ; le fond de la partie arrière 44 de la troisième chambre 43, comporte un orifice circulaire dans lequel passe librement un premier poussoir 49 cylindrique, solidaire du côté arrière 47 du deuxième piston 45 et orienté de préférence coaxialement avec le deuxième axe de symétrie de révolution 50 de la troisième chambre 43, qui est manoeuvré par des moyens de manoeuvre manuels des moyens de commande 8 (fig.1A et fig.1B) ; les moyens de manoeuvre manuels du premier poussoir 49 consistent de préférence à enfoncer le deuxième piston 45 dans la troisième chambre 43 ; un moyen de manoeuvre manuel est, par exemple, constitué par un levier articulé 51 autour d'un axe de rotation 52, perpendiculaire et décalé par rapport à l'axe de symétrie de révolution 50 de la troisième chambre 43 et comportant un plan de symétrie contenant l'axe de symétrie de révolution 50 et perpendiculaire à l'axe de rotation 52 ; les mouvements du levier articulé 51 sont limités par une butée 53 ; le côté avant 48 du deuxième piston 45 comporte une tige de commande 54 cylindrique de révolution, comportant une extrémité captive fixée sur le côté avant 48 du premier piston 45 et une extrémité libre 55 traversant librement un quatrième conduit 56 cylindrique de révolution et coaxial avec l'axe de symétrie de révolution 50 de la troisième chambre 43 ; le quatrième conduit 56 partant du fond de la partie avant 57 de la troisième chambre 43 est bordé par une surface tronconique de révolution coaxiale servant de guide à un troisième dispositif d'étanchéité de deuxième type 58 qui est neutralisé, en laissant passer le gaz dans le quatrième conduit 56, lorsque le deuxième piston 45 est, par exemple, en appui sur le fond de la partie arrière 44 de la troisième chambre 43, mais qui, lorsque le premier poussoir 49 est manoeuvré par le levier articulé 51, pénètre dans le quatrième conduit 56 et réalise l'étanchéité au gaz de la partie avant 57 de la troisième chambre 43 ; la tige de commande 54 se prolonge et son extrémité libre 55 pénètre dans un deuxième alésage 59 solidaire du corps de la troisième chambre 43 ; l'extrémité libre 55 de la tige de commande 54 interne au deuxième alésage 59 comporte un quatrième dispositif d'étanchéité de deuxième type 60 ; la course du deuxième piston 45 est délimitée en position arrière par la butée de son côté arrière 47 sur le fond de la partie arrière 44 de la troisième chambre 43 et elle est délimitée en position avant par la butée de l'extrémité libre 55 de la tige de commande 54 sur le fond 61 du deuxième alésage 59 ; en position avant du deuxième piston 45 le troisième dispositif d'étanchéité du deuxième type 58 obture le quatrième conduit 56.

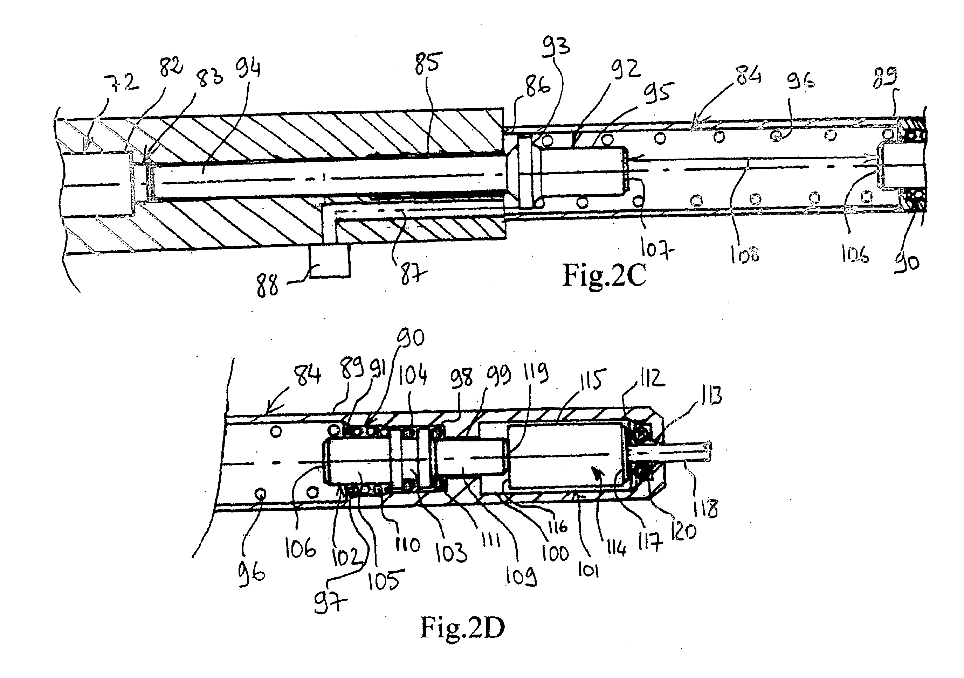

Un cinquième conduit 62 (fig.2B) comporte un orifice d'entrée débouchant dans le quatrième conduit 56 en aval de la position du quatrième dispositif d'étanchéité de deuxième type 58 et un orifice de sortie débouchant dans la partie arrière d'une quatrième chambre 64, qui est un moyen d'accumulation des gaz sous haute pression, de préférence cylindrique de révolution autour d'un axe de symétrie confondu avec celui du deuxième alésage 59 ; cette quatrième chambre comporte un clapet de décharge 65 qui se compose d'un corps de clapet 66, tubulaire cylindrique de révolution coaxial avec celui de la quatrième chambre 64 d'un diamètre extérieur de l'ordre du tiers de celui de la quatrième chambre 64, d'une tête de clapet 67, comportant un côté arrière plan et un côté avant 122 conique de révolution, d'un diamètre sensiblement double de celui du corps de clapet sur lequel il se raccorde et d'une base de corps de clapet 68 coulissant dans un troisième alésage 69, d'un diamètre du même ordre de grandeur que celui du corps de clapet 66 comportant un troisième dispositif d'étanchéité de premier type 70 coopérant avec la base de corps de clapet 68, le troisième alésage 69 étant pratiqué dans le fond de la partie avant 71 de la quatrième chambre 64 et débouchant dans une cinquième chambre 72 qui est une chambre de détente ; le côté arrière de la tête de clapet 67 est maintenue appliquée sur le fond de la partie arrière 63 de la quatrième chambre 64 par un premier ressort hélicoïdal 73 prenant appui sur le fond de la partie avant 71 de la quatrième chambre 64 ; le côté arrière de la tête du clapet 67 qui est circulaire comporte au voisinage de sa bordure une zone annulaire d'étanchéité coopérant avec un premier dispositif d'étanchéité de troisième type 74 solidaire du fond de la partie arrière 63 de la quatrième chambre 64 ; l'espace intérieur du corps de clapet 66 tubulaire constitue un sixième conduit 75 dont l'orifice d'entrée au niveau du côté arrière de la tête de clapet 67 est évasé et sensiblement conique ; la partie centrale de la tête de clapet 67 comporte un deuxième poussoir 76 cylindrique coaxial avec le deuxième alésage 59, comportant une extrémité libre 77 et une extrémité captive reliée au côté arrière de la tête de clapet 67 par des entretoises 78 intégrées dans l'ouverture évasée du sixième conduit 75 et coulissant dans un quatrième alésage 79 de révolution mettant en communication le fond de la partie arrière 63 de la quatrième chambre 64 avec le fond du deuxième alésage 59 ; un quatrième dispositif d'étanchéité de premier type 80, solidaire du quatrième alésage 79 assure l'étanchéité entre le deuxième alésage 59 et le sixième conduit 75 ; le deuxième poussoir 76 à un diamètre sensiblement inférieur à celui du deuxième alésage 59 et une longueur telle que, lorsque la tête de clapet 67 coopère avec le premier dispositif d'étanchéité de troisième type 74 pour isoler le sixième conduit 75 de la quatrième chambre 64, l'extrémité libre 77 du deuxième poussoir 76 débouche dans le fond du deuxième alésage 59 ; lorsque le deuxième piston 45 est en position avant, l'extrémité libre 55, de la tige de commande 54, appuie sur l'extrémité libre 77 du deuxième poussoir 76 et refoule la tête de clapet 67, en la décollant du fond de la partie arrière 63 de la quatrième chambre 64 en comprimant le premier ressort hélicoïdal 73, libérant l'ouverture du sixième conduit 75 ; la quatrième chambre 64 est mise en communication par l'intermédiaire du sixième conduit 75 avec la cinquième chambre 72 ; la cinquième chambre 72 est de forme cylindrique de révolution avec une partie arrière 81, dans laquelle débouche le sixième conduit 75 et une partie avant 82 de laquelle part un cinquième alésage 83 cylindrique de révolution et de préférence coaxial avec l'axe de symétrie de la cinquième chambre 72 ; la cinquième chambre 72 a un diamètre légèrement supérieur à celui de la base de corps de clapet 68 et de longueur sensiblement inférieure ; le cinquième alésage 83 (fig.2C) débouche dans une sixième chambre 84 cylindrique de révolution, coaxiale avec le cinquième alésage 83, d'un diamètre sensiblement égal à celui de la base de corps de clapet 68 et d'une longueur de l'ordre du double de celle de la quatrième chambre 64 ; le cinquième alésage 83 comporte une zone de décompression 85 d'un diamètre légèrement supérieur débouchant dans la sixième chambre 84 et d'une longueur correspondant sensiblement au tiers de la longueur du cinquième alésage 83 ; la sixième chambre 84 comporte une partie arrière 86 dans laquelle débouche le cinquième alésage 83 et de laquelle part au moins un septième conduit 87 communiquant avec l'atmosphère soit directement soit par l'intermédiaire d'une soupape anti-retour 88 ; la sixième chambre 84 comporte une partie avant 89 dans le fond de laquelle débouche une septième chambre, cylindrique de révolution coaxiale avec la sixième chambre 90 (fig.2C et fig.2D), communiquant avec la sixième chambre 84 par un sixième alésage 91 cylindrique de révolution, d'un diamètre sensiblement supérieur à celui du cinquième alésage 83 et inférieur à celui de la sixième chambre 90 ; le cinquième alésage 83 (fig.2C) sert de dispositif de guidage et de lancement à un marteau frappeur 92, constituant un moyen de frappe ; le marteau frappeur 92 est composé d'un corps de marteau 93, de faible épaisseur et d'un diamètre légèrement inférieur à celui de la sixième chambre dans laquelle il est situé, comportant du côté arrière tourné vers la partie arrière 86 de la sixième chambre 84 et un côté avant tourné vers la partie avant 89 de la sixième chambre 84 ; un troisième piston 94, cylindrique de révolution et coaxial avec le cinquième alésage 83, est solidaire du côté arrière du corps de marteau 93 et coulisse dans le cinquième alésage 83 ; le troisième piston 94 est d'un diamètre très légèrement inférieur à celui du cinquième alésage 83 afin d'obtenir une étanchéité suffisante pour en assurer la propulsion par les gaz ; une tête de frappe 95, cylindrique de révolution et coaxiale avec le troisième piston 94, est fixée sur la partie avant du corps de marteau 93 ; la tête de frappe 95 est d'un diamètre sensiblement plus grand que le diamètre du troisième piston 94 et d'une longueur de l'ordre du quart de la longueur du troisième piston 94 ; un deuxième ressort hélicoïdal 96 prenant appui sur la partie avant 89 de la sixième chambre 84 et sur le corps de marteau 93 appuie celui-ci sur le fond de la partie arrière 86 de la sixième chambre 84 en maintenant le troisième piston 94 enfoncé dans le cinquième alésage 83.

La septième chambre 90 (fig.2D) comporte une partie arrière 97 communiquant avec la sixième chambre 84 par le sixième alésage 91 et une partie avant 98 dont le fond comporte un septième alésage 99, cylindrique de révolution coaxial avec la septième chambre 90 et d'un diamètre sensiblement égal à celui du cinquième alésage 83, permettant à la septième chambre 90 de communiquer avec la partie arrière 100 d'une huitième chambre 101 ; la septième chambre 90 contient un dispositif interfaces 102 générateur d'onde de choc constitué d'un corps de dispositif interfaces 103, cylindrique de révolution susceptible de coulisser dans la septième chambre 90 et comportant un cinquième dispositif d'étanchéité de deuxième type 104 isolant la partie avant 98 de la partie arrière 97 de la septième chambre 90 ; le corps de dispositif interfaces 103 comprend un côté arrière tourné vers la partie arrière 97 de la septième chambre 90 et un côté avant tourné vers la partie avant 98 de la septième chambre 90 ; le côté arrière du corps de dispositif interfaces 103 comporte une enclume de frappe 105, de même diamètre que la tête de frappe 95, dont la longueur cumulée avec celle du corps de dispositif interfaces 103, est sensiblement égale à la longueur de la tête de frappe 95 cumulée avec celle du corps de marteau 93 ; la longueur du sixième alésage 91 est déterminée de manière que l'extrémité libre 106 de l'enclume de frappe 105 dépasse du fond de la partie avant 89 de la sixième chambre 84 et que la distance 108 (fig.2C) séparant l'extrémité libre 106 de l'enclume de frappe 105 de l'extrémité libre 107 de la tête de frappe 95 soit de l'ordre de grandeur de la longueur du troisième piston 94 diminuée d'une longueur suffisante pour assurer dans le cinquième alésage 83, un guidage résiduel du troisième piston 94 en fin de course ; le côté avant du corps de dispositif interfaces 103 comporte un premier dispositif de transfert de l'onde de choc 109, cylindrique de révolution et coaxial avec la septième chambre 90, d'un diamètre sensiblement égal à celui du troisième piston 94 et d'une longueur sensiblement égale à la longueur de l'enclume de frappe 105 ; la longueur du septième alésage 99 doit être suffisante pour assurer un guidage correct du dispositif interfaces 102 générateur d'onde de choc, mais elle doit permettre le dépassement du premier dispositif de transfert d'onde de choc 109 dans le fond de la partie arrière 100 de la huitième chambre 101 ; un troisième ressort hélicoïdal 110, prenant appui d'une part sur le fond de la partie arrière 97 de la septième chambre 90, d'autre part sur le côté arrière du corps de dispositif interfaces 103, appuie ce dernier sur un premier dispositif d'amortissement torique 111 s'appuyant d'une part sur la bordure de la partie avant du corps de dispositif interfaces 103 et entourant le premier dispositif de transfert d'onde de choc 109 et d'autre part sur le fond de la partie avant 98 de la septième chambre 90 ; la partie avant 112 de la huitième chambre 101 comporte un huitième alésage 113, cylindrique de révolution coaxial avec la huitième chambre 101 et de faible diamètre de l'ordre de la moitié du diamètre du troisième piston 94, communiquant avec l'extérieur ; la huitième chambre 101 contient une tête de guide d'onde de choc 115 d'un deuxième dispositif de guidage d'onde de choc 114, cylindrique de révolution de diamètre légèrement inférieur à celui de la huitième chambre 101, dont le côté arrière 116 est plan et le côté avant 117 est sensiblement plan et comporte, fixée perpendiculairement en son centre, une tige guide d'onde de choc 118 passant dans le huitième alésage 113 qui sert de guidage à la tête de guide d'onde de choc 115 du deuxième dispositif de guidage d'onde de choc 114 ; lorsque le dispositif générateur d'onde de choc mécanique à simple coup 1 (fig.1A et fig.1B) est en ordre de marche, la partie arrière 116 de la tête de guide d'onde de choc 115 est en contact avec l'extrémité libre 119 du premier dispositif de transfert d'onde de choc 109 et la partie avant 117 de la tête de guide d'onde de choc 115 vient en appui sur un deuxième dispositif d'amortissement torique 120 entourant la base de la tige guide d'onde de choc 118 et prenant appui d'une part sur le fond de la partie avant 112 de la huitième chambre 101 et d'autre part sur le côté avant 117 de la tête de guide d'onde de choc 115 ; l'importance du dépassement du premier dispositif de transfert d'onde de choc 109 et la longueur de la huitième chambre 101 est notamment déterminée en fonction de ces contraintes ; lorsque l'extrémité libre 119 du premier dispositif de transfert d'onde de choc 109 vient en appui sur le deuxième dispositif de transfert d'onde de choc 114, placé dans la huitième chambre 101, le dispositif interfaces 102 est légèrement repoussé vers la partie arrière 97 de la septième chambre 90 et le troisième ressort hélicoïdal 110 est légèrement comprimé ; la longueur et le diamètre de la tige guide d'onde de choc 118 sont généralement imposés par les conditions de mise en oeuvre ; le rendement maximum de l'onde de choc est obtenu lorsque le poids du marteau frappeur 92 (fig.2C) est égal au poids du deuxième dispositif de transfert d'onde de choc 114 ; le réglage de l'impédance peut se faire d'une part en agissant sur le diamètre de la tête de guide d'onde de choc 115 (fig.2D) d'autre part en agissant dans la mesure du possible sur la longueur du troisième piston 94 en tenant compte de ses impératifs de fonctionnement.

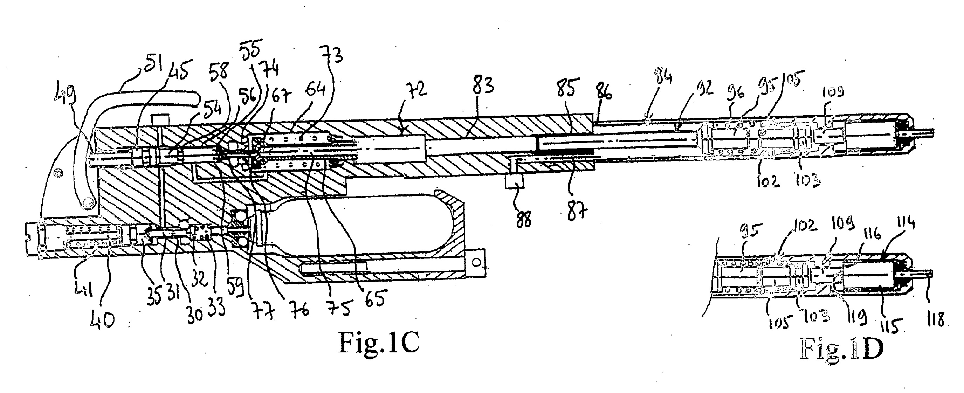

Lorsque la pression de gaz dans le deuxième conduit 30 (fig.1B) est en dessous de la pression nominale le deuxième ressort taré 41 repousse le premier piston 35 vers la partie arrière 38 de la deuxième chambre 36 ; la queue de soupape 31 s'enfonce dans le premier alésage 34 de la tête du premier piston 35 jusqu'à ce que la queue de soupape 31 en ayant touché le fond, la tête de soupape 32 d'alimentation ait repoussée comprimant le premier ressort taré 33 et libérant le passage du gaz en provenance du micro-conteneur de gaz 9 ; lorsque la pression de gaz dans le deuxième conduit 30 (fig.1C) remonte, le premier piston 35 s'enfonce en comprimant le deuxième ressort hélicoïdal 41 jusqu'à ce que, la tête de soupape 32 revenant en appui sur le deuxième dispositif d'étanchéité du premier type 29 sous l'action du premier ressort taré 33, la queue de soupape 31 perde le contact avec le fond du premier alésage 34 ; il est à noter que la détente du gaz étant adiabatique le gaz détendu est plus froid que la température ambiante et son réchauffement provoque une montée de pression qui est écrêtée par un recul supplémentaire du premier piston 35, comprimant le deuxième ressort taré 41 jusqu'à ce qu'il arrive éventuellement en contact avec la butée de piston 40. Le gaz injecté dans le deuxième conduit 30 (fig.1B et fig.2A) passe dans le troisième conduit 42 et de ce dernier dans la troisième chambre 43 et repousse le deuxième piston 45 en butée sur la partie arrière 44 de la troisième chambre 43 s'il n'y est déjà ; le troisième dispositif d'étanchéité de deuxième type 58 est alors situé à l'intérieur de la troisième chambre 43 ce qui permet au gaz de passer dans le quatrième conduit 56, puis dans le cinquième conduit 62 pour arriver dans la quatrième chambre 64 qui se remplit de gaz sous haute pression à une pression, par exemple, comprise entre quinze et trente bars ; la tête de clapet 67 est maintenue plaquée sur le premier dispositif d'étanchéité de troisième type 74 d'une part par le premier ressort hélicoïdal 73 et d'autre part par la haute pression de gaz ; lorsque la pression nominale est atteinte dans la quatrième chambre 64, le dispositif générateur d'onde de choc est prêt à fonctionner.

La génération d'une onde de choc commence par la manoeuvre du levier articulé 51 (fig.1C et fig.2B) qui enfonce le premier poussoir 49 qui repousse le deuxième piston 45 dans la troisième chambre 43 et la tige de commande 54 se déplace en translation dans la troisième chambre 43 jusqu'à ce que le troisième dispositif d'étanchéité de deuxième type 58 pénètre dans le quatrième conduit 56 et l'obture de manière à isoler la quatrième chambre 64 de la source d'alimentation en gaz haute pression ; puis l'enfoncement du premier poussoir 49 continuant l'extrémité libre 55 de la tige de commande 54 s'enfonce dans le deuxième alésage 59 jusqu'à venir enfoncer l'extrémité libre 77 du deuxième poussoir 76 ce qui provoque le décollement de la tête de clapet 67 du premier dispositif d'étanchéité de troisième type 74 et la compression du premier ressort hélicoïdal 73 ; le gaz s'engouffre dans le sixième conduit 75 et entre dans la cinquième chambre 72 et refoule avec violence le troisième piston 94 (fig.1C et fig.2C), qui était initialement en position enfoncée dans le cinquième alésage 83 grâce à l'action du deuxième ressort hélicoïdal 96 ; le marteau frappeur 92 est lancé à grande vitesse dans la sixième chambre 84 en comprimant le deuxième ressort hélicoïdal 96 et la tête de frappe 95 vient frapper l'enclume de frappe 105 (fig.1D et fig.2D) du dispositif interface 102 qui génère une onde de choc qui se propage successivement dans le corps de dispositif interfaces 103, puis dans le premier dispositif de transfert d'onde de choc 109 et qui est ensuite transmis à la partie arrière 116 la tête de guide d'onde de choc 115 pour se propager ensuite dans la tige guide d'onde de choc 118 jusqu'à l'objet à désintégrer.

En fin de course du troisième piston 94 dans le cinquième alésage 83 la zone de décompression 85 est progressivement découverte et le gaz commence à s'échapper dans la partie arrière 86 de la sixième chambre 84 et s'évacue vers l'extérieur par l'intermédiaire éventuel de la soupape anti-retour 88 et par le septième conduit 87 ; la pression en aval du troisième dispositif d'étanchéité de deuxième type 58 devient sensiblement égale à la pression atmosphérique ; le deuxième ressort hélicoïdal 96 repousse le marteau frappeur 92 et le troisième piston 94 dans le cinquième alésage 83 ; le premier ressort hélicoïdal 73 repousse le clapet de décharge 65 dans le fond de la partie arrière 63 (fig.1B et fig.2B) de la quatrième chambre 64 et rétablit l'étanchéité initiale à l'aide du premier dispositif d'étanchéité de troisième type 74 lorsque le deuxième poussoir 76 peut reprendre sa position initiale par relâchement de l'action sur le levier articulé 51 qui permet le déplacement du deuxième piston 45 vers la partie arrière 44 de la troisième chambre 43, sous l'action de la pression de gaz haute pression de la troisième chambre 43 ; le déplacement du deuxième piston 45 engendre le déplacement de la tige de commande 54 jusqu'à ce que la troisième étanchéité de deuxième type 58 échappe du quatrième conduit 56 ce qui permet l'alimentation de la quatrième chambre 64 en gaz ; la baisse de pression dans la troisième chambre 43 enclenche la baisse de pression dans le troisième et le second conduit 43 et 30 ce qui déclenche le cycle d'alimentation en gaz précédemment décrit.

Dans un perfectionnement de l'invention, la troisième chambre 43 (fig.2B) est reliée à l'extérieur par un deuxième clapet de décharge 121 calibré permettant d'éviter une surpression éventuelle liée au réchauffement du gaz après détente ou à une fuite au niveau de la tête de soupape 32 (fig.1A) et du premier dispositif d'étanchéité de premier type 19. A titre indicatif, le dispositif générateur d'onde de choc mécanique à simple coup 1 destiné à la désintégration de calculs urinaires possède une quatrième chambre 64 dont le volume est de préférence compris entre un et trois centimètres cubes et un marteau frappeur d'un poids de l'ordre de dix grammes.

Dans un perfectionnement de l'invention et pour des applications autres que celle concernant la destruction de calculs urinaires, il est nécessaire de procéder à la génération de trains d'ondes de choc successives ; pour cela il est intercalé entre le levier articulé 51 et le premier poussoir 49 un dispositif de déclenchement d'ondes de choc successives qui est activé par la manoeuvre du levier 51 et qui enchaîne la génération de plusieurs ondes de choc successives, par exemple, en nombre et à un rythme prédéterminé par manoeuvres successives du premier poussoir 49 sans qu'il soit nécessaire de relâcher le levier articulé 51.

## Revendications

1. Dispositif générateur d'ondes de choc mécaniques à simple coup (1) utilisant des moyens de frappe (2) mis en mouvement par des gaz et venant frapper à grande vitesse des moyens générateurs d'une onde de choc (3) qui est transmise par des moyens transfert d'onde de choc (4) pouvant être mis en contact direct ou indirect avec l'objet à désintégrer, **caractérisé en ce que** les moyens de frappe (2) sont mis en mouvement grâce à la détente d'un gaz sous haute pression de quinze à trente bars introduit, préalablement à chaque production d'une onde de choc dans des moyens d'accumulation (5) alimentés en gaz à partir de moyens de stockage autonomes (6) de gaz sous très haute pression de soixante dix à deux cents bars, grâce à des moyens de détente de gaz (7) à des moyens d'alimentation (25,26,30,42,56,62) et à des moyens d'étanchéification (19,37,46,60,70,74,80), le gaz stocké dans les moyens d'accumulation (5) étant libéré par la manoeuvre manuelle de moyens de commande (8) qui, dans un premier temps, rend étanche aux gaz, par un moyen d'étanchéification (58), la communication des moyens de stockage autonomes (6) et de détente (7) d'une part avec les moyens d'accumulation (5) d'autre part, puis, dans un deuxième temps, met en communication les moyens d'accumulation (5) et les moyens de frappe (2), le retour à la situation initiale des moyens de frappe (2) étant assuré par la libération d'énergie accumulée par des moyens mécaniques d'accumulation d'énergie (73,96) au cours du cycle de production de l'onde de choc, tandis que le retour à la situation initiale des moyens de commande (8) étant assuré par la haute pression des gaz restés au niveau des moyens de détente (7) et les moyens d'alimentation (25,26,30,42) correspondants.

2. Dispositif selon la revendication 1, **caractérisé en ce que** le gaz utilisé à la température et à la pression d'utilisation et chimiquement compatible avec son lieu d'utilisation, provient d'une bouteille de gaz sous pression constituant un moyen de stockage autonome qui est relié au dispositif générateur d'onde de choc mécanique à simple coup (1) par une tuyauterie et qui, par l'intermédiaire d'un détenteur constituant un moyen de détente, ramène la très haute pression à la pression d'utilisation.

3. Dispositif selon la revendication 2, **caractérisé en ce que** le gaz utilisé est stocké dans un micro-conteneur de gaz (9) constituant un moyen de stockage de gaz autonome (6) qui est directement intégré dans le dispositif générateur d'ondes de choc mécanique à simple coup (1) à l'aide d'un berceau (15) composé de deux demi-berceaux, un demi-berceau avant (16) comportant un fond muni d'un réceptacle calibré (18) comportant un dispositif de perforation (20), un demi-berceau arrière (21) étant composé d'un dispositif de maintien (22) coulissant, le dispositif de perforation (20) communiquant par l'intermédiaire d'un premier conduit (26) avec un dispositif de détente intégré (27) constituant un moyen de détente de gaz (7).

4. Dispositif selon la revendication 3, **caractérisé en ce que** le dispositif de détente intégré est constitué d'une première chambre (28) dans laquelle débouche le premier conduit (26) et d'où part un deuxième conduit (30) dans lequel coulisse librement une queue de soupape (31) dont la tête (32), située dans la première chambre (28), est repoussée par un premier ressort taré (33), l'extrémité libre de la queue de soupape (31) étant manoeuvrée par un premier piston cylindrique (35) servant de repoussoir de la queue de soupape (31), le premier piston (35) coulissant dans une deuxième chambre (36) servant de guide à un deuxième ressort taré (41) servant de repoussoir au piston (35).

5. Dispositif selon la revendication 4, **caractérisé en ce qu'**un moyen de commande (8) est composé d'une troisième chambre (43) dans laquelle débouche un troisième conduit (42) venant du deuxième conduit (30) et dans laquelle coulisse un deuxième piston (45) comportant un premier poussoir (49) qui permet d'enfoncer ledit piston dans la troisième chambre (43) à l'aide d'un levier articulé (51), le deuxième piston (45) comportant une tige de commande (54) présentant une extrémité libre (55) et traversant librement un quatrième conduit (56) comportant un troisième dispositif d'étanchéité de deuxième type (58) constitué par un joint torique placé dans une gorge pratiquée dans un cylindre de révolution qui obture le quatrième conduit (56), en amont d'un cinquième conduit (62) débouchant dans le quatrième conduit (56), lorsque le deuxième piston (45) est enfoncé dans la troisième chambre (43), l'extrémité libre (55) qui est engagée dans un deuxième alésage (59) venant alors en butée sur le fond (61) dudit alésage.

6. Dispositif selon la revendication 5, **caractérisé en ce qu'**un moyen d'accumulation (5) est constitué d'une quatrième chambre (64) dans laquelle débouche le cinquième conduit (62) comportant un clapet de décharge (65) composé d'un corps de clapet (66) tubulaire constituant un sixième conduit (75), d'une tête de clapet (67) creuse et d'une base de corps de clapet (68) coulissant dans un troisième alésage (69) débouchant dans une cinquième chambre (72), la tête de clapet (67) maintenant l'étanchéité grâce à un premier ressort hélicoïdal (73) constituant un moyen mécanique d'accumulation d'énergie, la tête de clapet (67) comportant un deuxième poussoir (76) ayant une extrémité libre (77) coulissant dans un quatrième alésage (79) et débouchant dans le fond du deuxième alésage (59), l'extrémité libre (77) du deuxième poussoir (76) étant repoussée par l'extrémité libre (55) de la tige de commande (54) lorsqu'elle est en butée sur le fond (61), le deuxième poussoir (76) refoulant la tête de clapet (67) en comprimant le premier ressort hélicoïdal (73) et libérant l'ouverture du sixième conduit (75) mettant la quatrième chambre (64) en communication avec la cinquième chambre (72).

7. Dispositif selon la revendication 6, **caractérisé en ce qu'**un moyen de frappe est constitué par la cinquième chambre (72) dans laquelle débouche le sixième conduit (75) et qui communique dans un cinquième alésage (83) débouchant dans une sixième chambre (84) comportant une zone de décompression (85) débouchant dans la sixième chambre (84) de laquelle part au moins un septième conduit (87) communiquant avec l'atmosphère, soit directement soit par l'intermédiaire d'une soupape anti-retour (88), le cinquième alésage (83) servant de dispositif de guidage et de lancement à un marteau frappeur (92) composé d'un corps de marteau (93), d'un troisième piston (94) coulissant dans le cinquième alésage (83), d'une tête de frappe (95) ayant une extrémité libre (107), un deuxième ressort hélicoïdal (96) constituant un moyen mécanique d'accumulation d'énergie appuyant sur le corps de marteau (93) afin de maintenir le troisième piston (94) enfoncé dans le cinquième alésage (83).

8. Dispositif selon la revendication 7, **caractérisé en ce qu'**un moyen générateur d'une onde de choc (3) est constitué par un dispositif interfaces (102) générateur d'onde de choc comportant un corps de dispositif interfaces (103) coulissant dans une septième chambre (90) communiquant avec la sixième chambre (84) par un sixième alésage (91), le corps de dispositif interfaces (103) comportant une enclume de frappe (105) traversant le sixième alésage (91) ayant une extrémité libre (106) située dans la sixième chambre (84), et un premier dispositif de transfert de l'onde de choc (109) ayant une extrémité libre (119) passant à travers un septième alésage (99) reliant la septième chambre à une huitième chambre (101) à l'intérieur de laquelle se trouve l'extrémité libre (119) du premier dispositif de transfert de l'onde de choc (109) qui est maintenue en contact avec le côté arrière (116) d'une tête de guide d'onde de choc (115) par un troisième ressort hélicoïdal (110).

9. Dispositif selon la revendication 8, **caractérisé en ce qu'**un moyen de transfert de l'onde de choc (4) est constitué par un deuxième dispositif de guidage d'onde de choc (114) composé de la tête de guide d'onde de choc (115) situé dans la huitième chambre (101) continuée par une tige guide d'onde de choc (118) passant dans un huitième alésage (113) servant de guidage à la tête de guide d'onde de choc (115) et communiquant avec l'extérieur.

10. Dispositif selon une ou plusieurs des revendications 6 à 9, **caractérisé en ce que** le gaz utilisé est du gaz carbonique stocké sous très haute pression de soixante dix à deux cents bars, la haute pression dans la quatrième chambre (64) étant de l'ordre de quinze à trente bars, le volume de la quatrième chambre (64) étant de l'ordre de un à trois centimètres cubes et le poids du marteau frappeur (92) est de l'ordre de dix grammes.

## Claims

1. Single-blow mechanical shockwave generation device (1) using striking means (2) caused to move by gasses and striking at great speed means (3) for generating a shockwave which is transmitted by shockwave transfer means (4) able to be brought into direct or indirect contact with the object to be disintegrated, **characterised in that** the striking means (2) are caused to move by virtue of the expansion of a gas under high pressure of fifteen to thirty bars introduced, prior to the production of each shockwave, into accumulation means (5) supplied with gas from self-contained storage means (6) for gas under extremely high pressure of seventy to two hundred bars by gas-expansion means (7), supply means (25, 26, 30, 42, 56, 62) and sealing means (19, 37, 46, 60, 70, 74, 80), the gas stored in the accumulation means (5) being released by manual operation of control means (8) which, in a first period, use a sealing means (58) to seal in a gas-tight manner the communication of the self-contained storage means (6) and the expansion means (7) on the one hand and the accumulation means (5) on the other hand, then, in a second period, allow communication between the accumulation means (5) and the striking means (2), the return of the striking means (2) to the initial condition being ensured by the release of energy accumulated by mechanical energy accumulation means (73, 96) during the production cycle of the shockwave, while the return of the control means (8) to the initial condition is ensured by the high pressure of the gasses which have remained at the expansion means (7) and by the corresponding supply means (25, 26, 30, 42).

2. Device as claimed in claim 1, **characterised in that** the gas used at the operating pressure and temperature and chemically compatible with its operating site comes from a pressurised gas bottle forming a self-contained storage means which is connected to the single-blow mechanical shockwave generation device (1) by tubing and which, by means of a pressure-reducing valve forming an expansion means, brings the extremely high pressure to the operating pressure.

3. Device as claimed in claim 2, **characterised in that** the gas used is stored in a gas micro-container (9) forming a self-contained gas storage means (6) which is directly integrated into the single-blow mechanical shockwave generation device (1) with the aid of a cradle (15) formed from two half-cradles, a front half-cradle (16) having a bottom provided with a calibrated receptacle (18) having a perforation device (20), a rear half-cradle (21) being formed from a sliding holding device (22), the perforation device (20) communicating by means of a first duct (26) with an integrated expansion device (27) forming a gas expansion means (7).

4. Device as claimed in claim 3, **characterised in that** the integrated expansion device is formed by a first chamber (28) into which issues the first duct (26) and from which leads a second duct (30) in which a valve stem (31) slides freely, the head (32) of which, located in the first chamber (28), is repelled by a first weighted spring (33), the free end of the valve stem (31) being moved by a first cylindrical piston (35) serving as a repelling device of the valve stem (31), the first piston (35) sliding in a second chamber (36) serving as a guide to a second weighted spring (41) serving as a repelling device to the piston (35).

5. Device as claimed in claim 4, **characterised in that** a control means (8) is formed from a third chamber (43) into which issues a third duct (42) coming from the second duct (30) and in which slides a second piston (45) having a first pusher (49) which makes it possible to force the said piston into the third chamber (43) with the aid of an articulated lever (51), the second piston (45) having a control rod (54) having a free end (55) and passing freely through a fourth duct (56) having a third sealing device (58) of a second type formed by an O-ring placed in a channel produced in a rotating cylinder which closes the fourth duct (56) upstream of a fifth duct (62) issuing into the fourth duct (56) when the second piston (45) is forced into the third chamber (43), the free end (55) which is engaged in a second bore (59) then coming into abutment on the base (61) of the said bore.

6. Device as claimed in claim 5, **characterised in that** an accumulation means (5) is formed by a fourth chamber (64) into which issues the fifth duct (62) comprising a discharge valve (65) formed from a tubular valve body (66) forming a sixth duct (75), a hollow valve head (67) and a valve body base (68) sliding in a third bore (69) issuing into a fifth chamber (72), the valve head (67) maintaining the sealing tightness by virtue of a first helical spring (73) forming a mechanical energy accumulation means, the valve head (67) having a second pusher (76) having a free end (77) sliding in a fourth bore (79) and issuing into the base of the second bore (59), the free end (77) of the second pusher (76) being repelled by the free end (55) of the control rod (54) when it is in abutment on the bottom (61), the second pusher (76) driving back the valve head (67) by compressing the first helical spring (73) and releasing the opening of the sixth duct (75) bringing the fourth chamber (64) into communication with the fifth chamber (72).

7. Device as claimed in claim 6, **characterised in that** a striking means is formed by the fifth chamber (72) into which issues the sixth duct (75) and which communicates in a fifth bore (83) issuing into a sixth chamber (84) comprising a decompression zone (85) issuing into the sixth chamber (84) from which leads at least one seventh duct (87) communicating with the atmosphere either directly or via a non-return valve (88), the fifth bore (83) serving as a guiding and launching device for a striking hammer (92) formed from a hammer body (93), a third piston (94) sliding in the fifth bore (83), a striking head (95) having a free end (107), a second helical spring (96) forming a mechanical energy accumulation means bearing against the hammer body (93) in order to keep the third piston (94) within the fifth bore (83).

8. Device as claimed in claim 7, **characterised in that** a shockwave generation means (3) is formed by a shockwave generation interface device (102) comprising an interface device body (103) sliding in a seventh chamber (90) communicating with the sixth chamber (84) by means of a sixth bore (91), the interface device body (103) having a striking anvil (105) passing through the sixth bore (91) having a free end (106) located in the sixth chamber (84), and a first shockwave transfer device (109) having a free end (119) passing through a seventh bore (99) connecting the seventh chamber to an eighth chamber (101) inside which is located the free end (119) of the first shockwave transfer device (109) which is kept in contact with the rear side (116) of a shockwave guiding head (115) by a third helical spring (110).

9. Device as claimed in claim 8, **characterised in that** a shockwave transfer means (4) is formed by a second shockwave guiding device (114) formed from the shockwave guiding head (115) located in the eighth chamber (101) continued by a shockwave guiding rod (118) passing into an eighth bore (113) serving to guide the shockwave guiding head (115) and communicating with the outside.

10. Device as claimed in one or several of claims 6 to 9, **characterised in that** the gas used is carbon dioxide stored under extremely high pressure of seventy to two hundred bars, the high pressure in the fourth chamber (64) being of the order of fifteen to thirty bars, the volume of the fourth chamber (64) being of the order of one to three cubic centimetres and the weight of the striking hammer (92) being of the order of ten grams.

## Patentansprüche

1. Vorrichtung zur Erzeugung mechanischer Stoßwellen mit einem Schlag (1) unter Benutzung von Anschlagmitteln (2), welche durch Gase in Bewegung gebracht werden und mit hoher Geschwindigkeit Mittel zur Erzeugung einer Stoßwelle (3) anschlagen, die durch Stoßwellenübertragungsmittel (4) übertragen wird, die mit dem zu zertrümmernden Objekt in direkten oder indirekten Kontakt gebracht werden können, **dadurch gekennzeichnet, dass** die Anschlagmittel (2) bewegt werden dank der Ausdehnung eines Gases unter hohem Druck von zwischen fünfzehn und dreißig Bar, das vor jeder Erzeugung einer Stoßwelle in Ansammlungsmittel (5) eingeführt wird, die aus autonomen Mitteln zur Lagerung (6) von Gas unter sehr hohem Druck zwischen siebzig und zweihundert Bar mit Gas versorgt werden, und dies dank Mitteln zur Ausdehnung von Gas (7), Versorgungsmitteln (25, 26, 30, 42, 56, 62) und Abdichtungsmitteln (19, 37, 46, 60, 70, 74, 80), wobei das in den Ansammlungsmitteln (5) gelagerte Gas durch die manuelle Betätigung von Steuermitteln (8) freigesetzt wird, was erstens durch ein Abdichtungsmittel (58) die Verbindung zwischen den autonomen Lagerungsmitteln (6) und den Ausdehnungsmitteln (7) einerseits und den Ansammlungsmitteln (5) andererseits gasdicht macht, dann zweitens die Ansammlungsmittel (5) und die Anschlagmittel (2) miteinander verbindet, wobei die Rückkehr zur Ausgangslage der Anschlagmittel (2) durch die Freisetzung von Energie gewährleistet wird, welche sich durch mechanische Mittel zur Ansammlung von Energie (73, 96) im Laufe des Erzeugungszyklus der Stoßwelle ansammelt, während die Rückkehr zur Ausgangslage der Steuermittel (8) durch den hohen Druck der Gase gewährleistet wird, die bei den Ausdehnungsmitteln (7) und den entsprechenden Versorgungsmitteln (25, 26, 30, 42) geblieben sind.

2. Vorrichtung nach Anspruch 1, **dadurch gekennzeichnet, dass** das Gas, das bei der Arbeitstemperatur und dem Arbeitsdruck benutzt wird und mit seinem Arbeitsort chemisch kompatibel ist, aus einer Gasflasche unter Druck stammt, die ein autonomes Lagerungsmittel bildet, das durch eine Rohrleitung mit der Vorrichtung zur Erzeugung einer mechanischen Stoßwelle mit einem Schlag (1) verbunden ist und das mittels eines Ausdehners, der ein Ausdehnungsmittel bildet, den sehr hohen Druck auf den Arbeitsdruck zurückbringt.

3. Vorrichtung nach Anspruch 2, **dadurch gekennzeichnet, dass** das Gas, das benutzt wird, in einem Gasmikrobehälter (9) gelagert wird, der ein autonomes Gaslagerungsmittel (6) bildet, das direkt in der Vorrichtung zur Erzeugung von mechanischen Stoßwellen mit einem Schlag (1) mithilfe einer Wiege (15) integriert ist, die aus zwei Halbwiegen zusammengesetzt ist, einer vorderen Halbwiege (16), umfassend einen Boden, der mit einem kalibrierten Behälter (18) versehen ist, der eine Perforationsvorrichtung (20) umfasst, einer hinteren Halbwiege (21), die aus einer gleitenden Haltungsvorrichtung (22) zusammengesetzt ist, wobei die Perforationsvorrichtung (20) durch eine erste Leitung (26) mit einer integrierten Ausdehnungsvorrichtung (27) in Verbindung steht, die ein Gasausdehnungsmittel (7) bildet.

4. Vorrichtung nach Anspruch 3, **dadurch gekennzeichnet, dass** die integrierte Ausdehnungsvorrichtung aus einer ersten Kammer (28) gebildet ist, in welche die erste Leitung (26) mündet und aus welcher eine zweite Leitung (30) austritt, in der ein Ventilschaft (31) frei gleitet, dessen Kopf (32), der sich in der ersten Kammer (28) befindet, von einer ersten vorgespannten Feder (33) zurückgestoßen wird, wobei das freie Ende des Ventilschafts (31) von einem ersten zylindrischen Kolben (35) betätigt wird, der als Zurückstoßer des Ventilschafts (31) dient, wobei der erste Kolben (35) in einer zweiten Kammer (36) gleitet, die als Führung für eine zweite vorgespannte Feder (41) dient, welche als Zurückstoßer für den Kolben (35) dient.

5. Vorrichtung nach Anspruch 4, **dadurch gekennzeichnet, dass** ein Steuermittel (8) aus einer dritten Kammer (43) gebildet ist, in die eine dritte Leitung (42) mündet, welche von der zweiten Leitung (30) kommt und in welcher ein zweiter Kolben (45) gleitet, der einen ersten Stößel (49) umfasst, der es ermöglicht, den Kolben mithilfe eines Gelenkhebels (51) in der dritten Kammer (43) zu versenken, wobei der zweite Kolben (45) eine Bedienungsstange (54) umfasst, die ein freies Ende (55) aufweist und eine vierte Leitung (56) frei durchquert, welche eine dritte Abdichtungsvorrichtung der zweiten Art (58) umfasst, bestehend aus einem O-Ring, der in einer Nut platziert ist, die in einem Drehzylinder eingesetzt wird, der die vierte Leitung (56) oberhalb einer fünften Leitung (62), die in die vierte Leitung (56) mündet, wobei, wenn der zweite Kolben (45) in der dritten Kammer (43) versenkt wird, verschließt, wobei das freie Ende (55), das mit einer zweiten Bohrung (59) in Eingriff steht, somit auf dem Boden (61) der Bohrung zum Anschlag kommt.

6. Vorrichtung nach Anspruch 5, **dadurch gekennzeichnet, dass** ein Ansammlungsmittel (5) aus einer vierten Kammer (64) gebildet ist, in welche die fünfte Leitung (62) mündet, umfassend ein Überströmventil (65), das aus einem tubulären Ventilkörper (66), der eine sechste Leitung (75) bildet, einem hohlen Ventilkopf (67) und einer Ventilkörperbasis (68), die in einer dritten Bohrung (69) gleitet, welche in eine fünfte Kammer (72) mündet, zusammengesetzt ist, wobei der Ventilkopf (67) die Dichtung dank einer ersten Schraubenfeder (73), die ein mechanisches Energieansammlungsmittel bildet, aufrecht erhält, wobei der Ventilkopf (67) einen zweiten Stößel (76) mit einem freien Ende (77) umfasst, das in einer vierten Bohrung (79) gleitet und in den Boden der zweiten Bohrung (59) mündet, wobei das freie Ende (77) des zweiten Stößels (76) durch das freie Ende (55) der Bedienungsstange (54) zurückgestoßen wird, wenn sie auf dem Boden (61) zum Anschlag kommt, wobei der zweite Stößel (76) den Ventilkopf (67) zurückhält, indem die erste Schraubenfeder (73) komprimiert wird und die Öffnung der sechsten Leitung (75) freigegeben wird, wodurch die vierte Kammer (64) mit der fünften Kammer (72) verbunden wird.

7. Vorrichtung nach Anspruch 6, **dadurch gekennzeichnet, dass** ein Anschlagmittel aus der fünften Kammer (72) gebildet ist, in welche die sechste Leitung (75) mündet und die in einer fünften Bohrung (83) verbunden ist, welche in eine sechste Kammer (84) mündet, umfassend eine Dekompressionszone (85), welche in die sechste Kammer (84) mündet, aus welcher mindestens eine siebte Leitung (87) austritt, die entweder direkt oder mittels eines Rückschlagventils (88) mit der Atmosphäre verbunden ist, wobei die fünfte Bohrung (83) als Führungs- und Wurfvorrichtung für einen Klopfhammer (92) dient, zusammengesetzt aus einem Hammerkörper (93), einem dritten Kolben (94), der in der fünften Bohrung (83) gleitet, einem Anschlagkopf (95) mit einem freien Ende (107), einer zweiten Schraubenfeder (96), die ein mechanisches Energieansammlungsmittel bildet, das auf den Hammerkörper (93) drückt, um den dritten Kolben (94), der in der fünften Bohrung (83) versenkt ist, zu halten.

8. Vorrichtung nach Anspruch 7, **dadurch gekennzeichnet, dass** ein Mittel zur Erzeugung einer Stoßwelle (3) aus einer Grenzflächenvorrichtung (102) zur Erzeugung einer Stoßwelle gebildet ist, umfassend einen Grenzflächenvorrichtungskörper (103), der in einer siebten Kammer (90) gleitet, die mit der sechsten Kammer (84) durch eine sechste Bohrung (91) in Verbindung steht, wobei der Grenzflächenvorrichtungskörper (103) einen Anschlagamboss (105) umfasst, der die sechste Bohrung (91) durchquert, die ein freies Ende (106) aufweist, das sich in der sechsten Kammer (84) befindet, und wobei eine erste Vorrichtung zur Übertragung der Stoßwelle (109) ein freies Ende (119) aufweist, das durch eine siebte Bohrung (99) verläuft, welche die siebte Kammer mit einer achten Kammer (101) verbindet, innerhalb welcher sich das freie Ende (119) der ersten Vorrichtung zur Übertragung der Stoßwelle (109) befindet, die mit der Hinterseite (116) eines Stoßwellenleiterkopfs (115) durch eine dritte Schraubenfeder (110) in Kontakt gehalten wird.

9. Vorrichtung nach Anspruch 8, **dadurch gekennzeichnet, dass** ein Mittel zur Übertragung der Stoßwelle (4) aus einer zweiten Stoßwellenleitungsvorrichtung (114) gebildet ist, die sich aus dem Stoßwellenleiterkopf (115) zusammensetzt, der sich in der achten Kammer (101) befindet, die durch eine Stoßwellenleiterstange (118) fortgesetzt wird, welche durch eine achte Bohrung (113) verläuft, die als Führung für den Stoßwellenleiterkopf (115) dient und mit außen in Verbindung steht.

10. Vorrichtung nach einem oder mehreren der Ansprüche 6 bis 9, **dadurch gekennzeichnet, dass** das Gas, das benutzt wird, Kohlendioxid ist, das unter sehr hohem Druck von siebzig bis zweihundert Bar gelagert wird, wobei der hohe Druck in der vierten Kammer (64) im Bereich von fünfzehn bis dreißig Bar liegt, wobei das Volumen der vierten Kammer (64) im Bereich von einem bis drei Kubikzentimetern liegt und das Gewicht des Anschlaghammers (92) im Bereich von zehn Gramm liegt.
